# EUROPÄISCHE PATENTANMELDUNG

(11) **EP 4 252 715 A1**
(43) Veröffentlichungstag der Anmeldung: **04.10.2023**
(21) Anmeldenummer: 22164792.8
(22) Anmeldetag: 28.03.2022
(51) Int. Cl.: A61F 2/28, A61F 2/78, A61B 17/74, A61F 2/30, A61F 2/36, A61B 17/76

(54) **FEMURIMPLANTAT UND FEMURIMPLANTAT-SET**

(71) Anmelder: Grundei, Hannu, 23564 Lübeck (DE); Grundei, Ulla, 23564 Lübeck (DE)
(72) Erfinder: Grundei, Hans, 23564 Lübeck (DE)
(74) Vertreter: Fuchs Patentanwälte Partnerschaft mbB

(57) **Zusammenfassung**

Ein Femurimplantat (104; 204) zum Bilden einer Femur-Komponente (201) eines transkutanen Implantats (200), insbesondere zur Verwendung mit einem Exo-Kniegelenk, umfasst eine Stielteil-Anordnung (104; 204), die in einem Femurknochenstumpf (F) verankerbar ist. Die Stielteil-Anordnung (104; 204) weist wenigstens eine Anbringungsöffnung (142; 242) auf, die in einem proximalen Bereich (140; 226) der Stielteil-Anordnung (104; 204) angeordnet ist und die dazu ausgebildet ist, ein Anbringen einer Schenkelhals-Endoprothese (108; 208) an der Stielteil-Anordnung (104; 204) zu gestatten, wenn die Stielteil-Anordnung (104; 204) in einem Femurknochenstumpf (F) verankert ist. Ein Femurimplantat-Set (100) umfasst ein Femurimplantat der vorgestellten Art.

## Beschreibung

Die Anmeldung betrifft ein Femurimplantat zum Bilden einer Femur-Komponente eines transkutanen Implantats, insbesondere zur Verwendung mit einem Exo-Kniegelenk. Die Anmeldung betrifft ferner ein Femurimplantat-Set.

### Technischer Hintergrund

Beinprothesen kommen typischerweise nach Verlust eines Beinteils, etwa infolge von Krankheit, Unfall oder Gewalteinwirkung, zum Einsatz. Sie dienen dazu, dem Betroffenen Funktionen des verlorenen Körperteils zumindest teilweise wieder zur Verfügung zu stellen oder zu ersetzen.

Um nach einem Beinteilverlust eine Bein- oder Knieprothese am Oberschenkelstumpf anzubringen, sind unter Anderem transkutane Implantate bekannt. Diese umfassen eine Femurkomponente, die in dem Femurknochenstumpf des Patienten befestigt wird. Unterhalb der Femurkomponente ist daran eine Anordnung von Brücken- und Anbindungselementen vorgesehen, die durch die Haut des Patienten bis an die Außenseite des Körpers ragt und dort die Befestigung einer äußeren, sogenannten Exo-, Knieprothese gestattet.

Um die Femurkomponente in dem Femurknochenstumpf zu befestigen, umfasst diese bei bekannten Systemen ein Femurimplantat, das im Markraum des Femurknochenstumpfs verankert wird. Um eine stabile Fixierung und eine günstige Übertragung von Belastungskräften zwischen dem Knochen und dem Femurimplantat zu erzielen, ist das Femurimplantat entsprechend dem Markraum im Wesentlichen stielförmig ausgebildet; es wird daher auch "Stielteil" genannt. Vor dem Einbringen des Stielteils wird der Femurknochenstumpf, sofern erforderlich, zudem auf eine dazu vorgesehene Länge gekürzt.

Bei einigen Patienten hat sich ein erhöhtes Bruchrisiko im Femurskelett oberhalb des Femurimplantats gezeigt. Besonders betroffen ist dabei die Region des Schenkelhalses. Die Behandlung besteht in solchen Fällen herkömmlich in dem Implantieren einer Schenkelhalsschraube. Diese wird dabei typischerweise von lateral oberhalb des Femurimplantats und im Wesentlichen parallel zu einer Achse des Schenkelhalses in den Knochen eingebracht.

Zudem ist bekannt, bei Patienten mit Femurimplantat im Fall einer später erforderlich werdenden Hüftoperation anstelle eines sonst herkömmlich in den Femur eingebrachten Stiels eines künstlichen Hüftgelenks ein außerhalb am Femur befestigtes Plattenimplantat zu verwenden.

Ausgehend davon ist eine Technik wünschenswert, die für Patienten mit einem Femurimplantat eine verbesserte Stützung des Schenkelhalses und eine verbesserte Anbringung eines künstlichen Hüftgelenks ermöglicht.

### Abriss der Erfindung

Es werden daher ein Femurimplantat gemäß Anspruch 1 und ein Femurimplantat-Set gemäß Anspruch 12 vorgestellt.

Gemäß einem ersten Aspekt wird ein Femurimplantat zum Bilden einer Femur-Komponente eines transkutanen Implantats, insbesondere zur Verwendung mit einem Exo-Kniegelenk, vorgestellt. Das Femurimplantat umfasst eine Stielteil-Anordnung, die in einem Femurknochenstumpf verankerbar ist. Die Stielteil-Anordnung weist wenigstens eine Anbringungsöffnung auf, die in einem proximalen Bereich der Stielteil-Anordnung angeordnet ist und die dazu ausgebildet ist, ein Anbringen einer Schenkelhals-Endoprothese an der Stielteil-Anordnung zu gestatten, wenn die Stielteil-Anordnung in einem Femurknochenstumpf verankert ist.

Die Schenkelhals-Endoprothese kann eine Schenkelhalsschraube zur Schenkelhalsstabilisierung umfassen oder als solche ausgebildet sein. Die Schenkelhals-Endoprothese kann einen Schaft eines künstlichen Hüftgelenks umfassen oder als solcher ausgebildet sein. Die Schenkelhals-Endoprothese kann eine Kombination aus einer Schenkelhalsschraube und einem Schaft eines künstlichen Hüftgelenks umfassen oder als solche ausgebildet sein. In einer bevorzugten Ausführung ist die Anbringungsöffnung dazu ausgebildet, eine Übertragung von Stützkräften zwischen der Schenkelhals-Endoprothese und der Stielteil-Anordnung zu gestatten. Eine Stützwirkung mittels der Schenkelhals-Endoprothese und der Stielteil-Anordnung kann dadurch begünstigt werden.

In einer weiteren bevorzugten Ausführung ist die Anbringungsöffnung dazu ausgebildet, ein Einbringen der Schenkelhals-Endoprothese wenigstens teilweise durch die Anbringungsöffnung, insbesondere aus lateraler Richtung, zu gestatten. Dies kann ein Einbringen der Schenkelhals-Endoprothese zu einem beliebigen Zeitpunkt nach erfolgter Verankerung der Stielteil-Anordnung in einem Femurknochenstumpf begünstigen.

Die Stielteil-Anordnung kann einen Stielteil-Grundkörper umfassen, der sich zumindest im Wesentlichen über eine Gesamtlänge der Stielteil-Anordnung erstreckt. Die Anbringungsöffnung kann dabei in einem proximalen Bereich des Stielteil-Grundkörpers angeordnet sein.

Alternativ kann die Stielteil-Anordnung wenigstens einen Stielteil-Grundkörper und wenigstens ein Brückenmodul, das mit dem Stielteil-Grundkörper in einer Längserstreckungsrichtung der Stielteil-Anordnung verbindbar ist, umfassen. Die Anbringungsöffnung kann dabei in dem Brückenmodul angeordnet sein.

Das Brückenmodul kann derart ausgebildet sein, dass die Anbringungsöffnung in Bezug auf einen Schenkelhals des Femurknochenstumpfs in einem vorgesehenen Bereich angeordnet ist, wenn die Stielteil-Anordnung gemäß einer vorgesehenen Position in Bezug auf ein distales Ende des Femurknochenstumpfs verankert ist.

Die Lage und Position des Brückenmoduls und des Stielteil-Grundkörpers können relativ zueinander fixierbar sein. Beispielsweise können eine zusätzliche laterale Fixierungsbohrung und ein/e darin einbringbare Fixierungsschraube oder Fixierungsstift vorgesehen sein. Hierdurch kann eine Relativbewegung zwischen Stielteil-Grundkörper und Brückenmodul nach einem Zusammenfügen sicher verhindert werden.

Zusätzlich oder alternativ können die Lage und Position der Schenkelhals-Endoprothese und der Stielteil-Anordnung relativ zueinander fixierbar sein. Beispielsweise können im proximalen Bereich der Stielteilanordnung, insbesondere im proximalen Bereich des Brückenmoduls, parallel zur Längserstreckungsrichtung, insbesondere koaxial, eine zusätzliche Fixierungsbohrung und ein/e darin einbringbare Fixierungsschraube oder Fixierungsstift vorgesehen sein. Hierdurch kann eine Relativbewegung zwischen Schenkelhals-Endoprothese und Stielteil-Anordnung nach einem Zusammenfügen sicher verhindert werden.

Die Stielteil-Anordnung kann somit in bevorzugten Ausführungsformen einen modularen, mehrteiligen Aufbau haben. Alternativ kann die Stielteil-Anordnung einen einstückigen Aufbau haben und in verschiedenen Längen bereitgestellt werden.

Die Stielteil-Anordnung kann in einer Längserstreckungsrichtung eine Größe im Bereich von 120 Millimeter bis 200 Millimeter, bevorzugt im Bereich von 140 Millimeter bis 180 Millimeter, aufweisen.

Die Anbringungsöffnung kann zur Übertragung von Belastungskräften zwischen einer Schenkelhals-Endoprothese und der Stielteil-Anordnung ausgebildet sein. Die Anbringungsöffnung kann eine Bohrung umfassen, die sich durch die Stielteilanordnung erstreckt. Die Anbringungsöffnung kann in Bezug auf eine Längsachse der Stielteil-Anordnung derart ausgerichtet sein, dass sie ein Anbringen der Schenkelhals-Endoprothese an der Stielteil-Anordnung zumindest im Wesentlichen gemäß einem Schenkelhalswinkel (CCD-Winkel) gestattet.

Die Anbringungsöffnung kann in Bezug auf die Längsachse der Stielteil-Anordnung in einem Winkel im Bereich von 105 Grad bis 155 Grad, vorzugsweise 115 Grad bis 150 Grad, bevorzugt 130 Grad bis 145 Grad ausgerichtet sein, vorzugsweise im Bereich von 135 Grad bis 140 Grad.

Insbesondere durch den möglichen modularen Aufbau kann so individuell die längen- und/oder winkelmäßig passende Anordnung für den jeweiligen Patienten bereitgestellt werden. Somit wird ermöglicht, dass das Femurimplantat dem Patienten so implantiert werden kann, dass der Scheitelpunkt des Winkels zwischen Anbringungsöffnung und Längsachse der Stielteil-Anordnung im Wesentlichen oder exakt an dem Scheitelpunkt des Schenkelhalswinkels (CCD-Winkels) angeordnet werden kann.

Die Anbringungsöffnung kann zum Durchführen von wenigstens einem Teil der Schenkelhals-Endoprothese durch die Stielteil-Anordnung, insbesondere von lateral, ausgebildet sein.

Die Anbringungsöffnung kann einen Durchmesser im Bereich von 6 Millimeter bis 15 Millimeter, bevorzugt im Bereich von 8 Millimeter bis 12 Millimeter, aufweisen.

Gemäß einem weiteren Aspekt wird ein Femurimplantat-Set vorgestellt. Das Femurimplantat-Set umfasst wenigstens ein Femurimplantat der hier vorgestellten Art.

Zusätzlich oder alternativ umfasst das Femurimplantat-Set wenigstens eine Schenkelhals-Endoprothese, die mittels der Anbringungsöffnung der Stielteil-Anordnung des wenigstens einen Femurimplantats an der Stielteil-Anordnung anbringbar ist.

Zusätzlich oder alternativ umfasst die Stielteil-Anordnung des Femurimplantats des Femurimplantat-Sets wenigstens einen Stielteil-Grundkörper und wenigstens ein Brückenmodul, das mit dem Stielteil-Grundkörper in einer Längserstreckungsrichtung der Stielteil-Anordnung verbindbar ist. Das Femurimplantat-Set umfasst dabei eine Mehrzahl von Brückenmodulen, die wahlweise mit dem wenigstens einen Stielteil-Grundkörper verbindbar sind und die jeweils eine Anbringungsöffnung und einen Brückenmodul-Verbindungsabschnitt zum Verbinden des Brückenmoduls mit dem Stielteil-Grundkörper aufweisen, wobei die Brückenmodule bezüglich wenigstens eines aus einem Abstand zwischen der Anbringungsöffnung und dem Brückenmodul-Verbindungsabschnitt, einer Ausrichtung der Anbringungsöffnung in Bezug auf eine Längsachse der Stielteil-Anordnung und eines Durchmessers der Anbringungsöffnung verschieden sind.

Zusätzlich oder alternativ umfasst die Stielteil-Anordnung des Femurimplantats des Femurimplantat-Sets wenigstens einen Stielteil-Grundkörper und wenigstens ein Brückenmodul, das mit dem Stielteil-Grundkörper in einer Längserstreckungsrichtung der Stielteil-Anordnung verbindbar ist. Das Femurimplantat-Set umfasst dabei eine Mehrzahl von Stielteil-Grundkörpern, die wahlweise mit dem wenigstens einen Brückenmodul verbindbar sind, wobei die Stielteil-Grundkörper bezüglich einer Größe in einer Längserstreckungsrichtung der Stielteil-Anordnung verschieden sind.

Die Schenkelhals-Endoprothese kann zum Verstärken eines Schenkelhalses und/oder zum Anbringen wenigstens eines Teils einer Hüft-Endoprothese an der Schenkelhals-Endoprothese ausgebildet sein, wenn das Femurimplantat in einem Femurknochenstumpf verankert ist.

Insbesondere kann die Schenkelhals-Endoprothese eine Schenkelhalsschraube umfassen. Die Anbringungsöffnung kann dabei eine Bohrung umfassen, und die Schenkelhalsschraube kann in einem medialen Abschnitt ein Gewinde und in einem lateralen Abschnitt ein Zylinderprofil aufweisen.

Merkmale, Vorteile und technische Effekte, die vorstehend lediglich in Bezug auf das Femurimplantat beschrieben wurden, können entsprechend für das Femurimplantat-Set gelten und andersherum.

### Kurze Beschreibung der Zeichnungen

Weitere Merkmale, Vorteile und Ziele der Erfindung werden aus den Zeichnungen und der ausführlichen Beschreibung deutlich. Es zeigen:
- Fig. 1 bis 3: Femurimplantat-Sets umfassend jeweils wenigstens ein Femurimplantat, gemäß verschiedenen Beispielen;
- Fig. 4: ein Brückenmodul und eine Schenkelhals-Endoprothese eines Femurimplantat-Sets, gemäß einem weiteren Beispiel;
- Fig. 5A: einen Femurknochenstumpf mit darin verankertem Stielteil herkömmlicher Art; und
- Fig. 5B: einen Femurknochenstumpf mit Bezugsmerkmalen zum Auswählen bzw. Konfigurieren einer Stielteil-Anordnung, gemäß einem Beispiel.

### Ausführliche Beschreibung

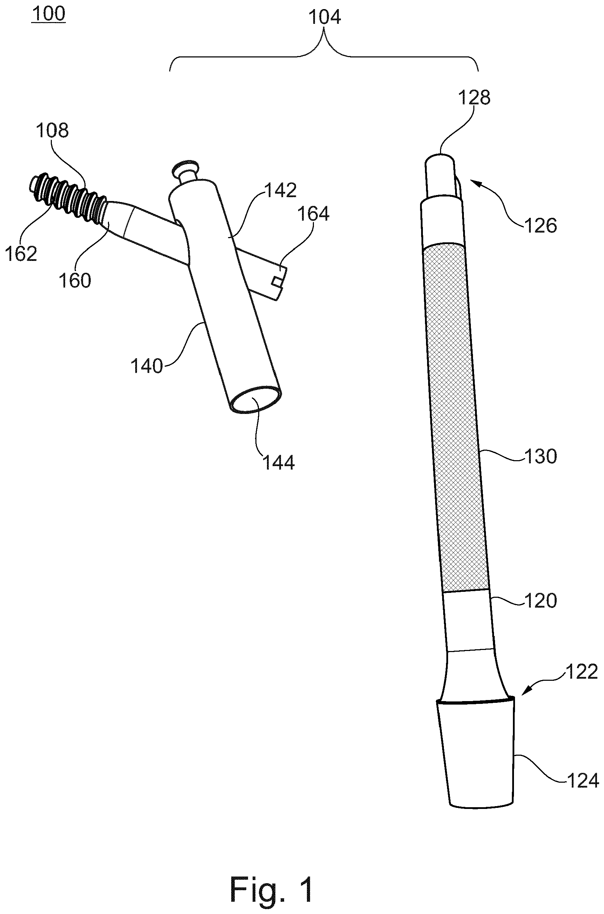

Fig. 1 zeigt schematisch und exemplarisch ein Femurimplantat-Set 100. Das Femurimplantat-Set 100 umfasst ein Femurimplantat 104 in Form einer Stielteil-Anordnung zum Bilden einer Femurkomponente eines transkutanen Implantats, das besonders zur Verwendung mit einem Exo-Kniegelenk vorgesehen ist. Das Femurimplantat 104 ist für diesen Zweck zur Implantation in den Markraum eines Femurknochenstumpfs ausgebildet, ähnlich dem Stielteil herkömmlicher Systeme.

In dem gezeigten Beispiel umfasst die Stielteil-Anordnung 104 einen Stielteil-Grundkörper 120 und ein Brückenmodul 140. Der Stielteil-Grundkörper 120 und das Brückenmodul 140 sind in einer Längserstreckungsrichtung der Stielteil-Anordnung 104 verbindbar. Dazu weist das Brückenmodul 140 an einem distalen Ende einen Brückenmodul-Verbindungsabschnitt 144 auf. Dieser ist mit einem Grundkörper-Verbindungsabschnitt 128, der in einem proximalen Bereich 126 des Stielteil-Grundkörpers 120 angeordnet ist, verbindbar. Im verbundenen Zustand ist der Stielteil-Grundkörper 120 in einem distalen Bereich der Stielteil-Anordnung 104 und das Brückenmodul 140 in einem proximalen Bereich der Stielteil-Anordnung 104 angeordnet.

In einem distalen Bereich 122 des Stielteil-Grundkörpers weist dieser einen Verbindungskonus 124 auf. Der Verbindungskonus 124 dient zum Anbringen einer Anordnung von Brücken- und Anbindungselementen, die durch die Haut des Patienten ragt und die Befestigung einer Bein- bzw. Knieprothese gestattet, wenn die Stielteil-Anordnung 104 in einem Femurknochenstumpf verankert ist. Um ein Verwachsen der Stielteil-Anordnung 104 mit dem Knochen zu begünstigen, weist der Steilteil-Grundkörper 120 an seiner Mantelfläche eine spongiöse Oberflächenstrukturierung 130 auf, hier eine dreidimensionale interkonnektierende Raumstruktur.

Die voranstehend beschriebenen Funktionen der Stielteil-Anordnung 104 ähneln im Wesentlichen denen eines herkömmlichen, typischerweise einstückig ausgebildeten Stielteil zum Bilden einer Femurkomponente eines transkutanen Implantats.

Die Stielteil-Anordnung 104 weist im Bereich des Brückenmoduls 140 eine Anbringungsöffnung 142 auf. In dem gezeigten Beispiel ist die Anbringungsöffnung 142 als Bohrung ausgestaltet, die sich schräg zur Längsachse der Stielteil-Anordnung 104 durch das Brückenmodul 140 erstreckt.

Das Femurimplantat-Set 100 umfasst außerdem eine Schenkelhals-Endoprothese 108. Diese ist mittels der Anbringungsöffnung 142 an der Stielteil-Anordnung 104 anbringbar.

Das Femurimplantat-Set 100 gestattet mittels der Anbringungsöffnung 142 das Anbringen einer Schenkelhals-Endoprothese 108 an der Stielteil-Anordnung 104. Das begünstigt die Schaffung einer zusammenhängenden Struktur stützender Implantate im Inneren des Knochens. Die Anbringungsöffnung 142 gestattet dabei zudem ein vorteilhaftes Platzieren der Schenkelhals-Endoprothese 108, beispielsweise entlang einer zentralen Achse des Schenkelhalses. Die Anbringungsöffnung 142 gestattet überdies ein Einbringen der Schenkelhals-Endoprothese 108 aus lateraler Richtung in den Schenkelhals nach einer Verankerung der Stielteil-Anordnung 104 im Femurknochenstumpf.

Beim Einbringen der Stielteil-Anordnung 104 in den Knochen erfolgt beispielsweise bereits eine geeignete Platzierung und Ausrichtung der Anbringungsöffnung 142. Bei einem später auftretenden Erfordernis beispielsweise einer Verstärkung des Schenkelhalses und/oder einer Abstützung eines künstlichen Hüftgelenks lässt sich eine entsprechende Schenkelhals-Endoprothese, insbesondere eine Schenkelhalsschraube oder ein Schaft eines künstlichen Hüftgelenks, von außen, insbesondere von lateral, durch die Anbringungsöffnung 142 einsetzen und an der Stielteil-Anordnung 104 anbringen. Eine ursprünglich erfolgte Verankerung der Stielteil-Anordnung 104 in dem Knochen braucht dabei beispielsweise nicht gelöst zu werden.

In dem gezeigten Beispiel von Fig. 1 umfasst die Schenkelhals-Endoprothese eine Schenkelhalsschraube 160. Diese weist in einem Bereich, der einem medialen Ende der Schenkelhalsschraube 160 entspricht, ein Gewinde 162 auf. Ein dem Gewinde 162 gegenüber liegender lateraler Abschnitt der Schenkelhalsschraube 160 weist dagegen ein Zylinderprofil 164 auf.

Das Zylinderprofil 164 gestattet in Verbindung mit der Bohrung 142 ein Eindrehen des Gewindes 162 in den Schenkelhals durch Drehen der Schenkelhalsschraube 160 gegenüber der Stielteil-Anordnung 104. Außerdem gestattet das Zylinderprofil 164 in Verbindung mit der Bohrung 142 eine Übertragung von Stützkräften zwischen der Schenkelhalsschraube 160 und der Stielteil-Anordnung 104 mittels Formschluss nach erfolgtem Einbringen der Schenkelhalsschraube 160.

In einigen Beispielen gestattet das Gewinde 162 der Schenkelhalsschraube 160 zudem ein Anbringen eines künstlichen Hüftgelenks, das dabei mittels der Schenkelhalsschraube an die Stielteil-Anordnung 104 angebunden und gestützt ist.

Die modulare Ausbildung der Stielteil-Anordnung 104 in dem Beispiel aus Fig. 1 begünstigt eine Anpassung der Stielteil-Anordnung 104 an verschiedene Femurgeometrien und Anwendungen.

Beispielsweise ist der Stielteil-Grundkörper 120 wahlweise mit verschiedenen Brückenmodulen 140 verbindbar, die sich bezüglich eines Abstands zwischen der Anbringungsöffnung 142 und dem Brückenmodul-Verbindungsabschnitt 144 unterscheiden. Dies gestattet eine Anpassung der Stielteil-Anordnung 104 an verschiedene Knochenlängen, beispielsweise gemäß einem Abstand zwischen einem vorgesehenen Bereich zur Implantation des Stielteil-Grundkörpers 120 in Bezug auf ein distales Ende des Femurknochenstumpfs und einem vorgesehenen Bereich für die Anbringungsöffnung 142 in Bezug auf den Schenkelhals.

Zusätzlich oder alternativ unterscheiden sich in weiteren Beispielen verschiedene Brückenmodule 140 bezüglich einer Ausrichtung der Anbringungsöffnung 142 relativ zur Längsachse der Stielteil-Anordnung 104. Dies gestattet beispielsweise eine Anpassung der Stielteil-Anordnung 104 an verschiedene Schenkelhalswinkel. Ein Schenkelhalswinkel liegt typischerweise im Bereich zwischen 135 Grad und 145 Grad. Verschiedene Brückenmodule 140 weisen dabei beispielsweise verschiedene Ausrichtungen der Anbringungsöffnung 142 im Bereich zwischen 130 Grad und 150 Grad auf.

Zusätzlich oder alternativ unterscheiden sich in weiteren Beispielen verschiedene Brückenmodule 140 bezüglich eines Durchmessers der Anbringungsöffnung 142. Dies gestattet beispielsweise eine Anpassung der Stielteil-Anordnung 104 an verschiedene Größen der Schenkelhals-Endoprothese gemäß verschiedenen Anwendungen. Verschiedene Brückenmodule 140 weisen dabei beispielsweise verschiedene Durchmesser der Anbringungsöffnung 142 im Bereich zwischen 6 und 15 Millimeter auf.

Zusätzlich oder alternativ ist in einigen Beispielen ein Brückenmodul 140 mit verschiedenen Stielteil-Grundkörpern 120 verbindbar, die sich bezüglich einer Länge in der Längserstreckungsrichtung der Stielteil-Anordnung, beispielsweise bezüglich eines Abstands zwischen dem Verbindungskonus 124 und dem Grundkörper-Verbindungsabschnitt 128 unterscheiden. Dies gestattet eine Anpassung der Stielteil-Anordnung 104 an verschiedene Knochenlängen, beispielsweise gemäß einem Abstand zwischen einem distalen Ende des Femurknochenstumpfs und einem vorgesehenen Bereich für den Grundkörper-Verbindungsabschnitt 128 in Bezug auf eine proximale Topologie des Femurknochenstumpfs.

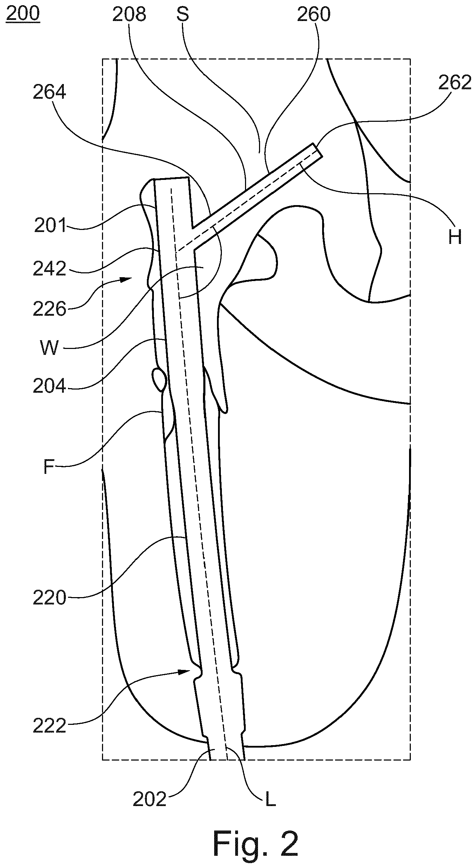

Fig. 2 zeigt schematisch und exemplarisch ein transkutanes Implantat 200, das in einem Oberschenkelstumpf verankert ist. Das transkutane Implantat 200 umfasst eine Femurkomponente 201 und eine daran angebrachte Anordnung 202 von Brücken- bzw. Anbindungselementen, die durch die Haut des Oberschenkelstumpfs ragt, insbesondere zum Befestigen einer Exo-Knieprothese oder einer Beinteilprothese. Die Femurkomponente 201 umfasst ein Femurimplantat-Set mit einer Stielteil-Anordnung 204 und einer Schenkelhals-Endoprothese 208, die mittels einer Anbringungsöffnung 242 in einem proximalen Bereich 226 der Stielteil-Anordnung 204 mit dieser verbunden ist.

Für die Stielteil-Anordnung 204 und die Schenkelhals-Endoprothese 208 gilt das voranstehend zu der Stielteil-Anordnung 104 und der Schenkelhals-Endoprothese 108 in Fig. 1 Gesagte entsprechend, soweit sich aus dem Nachfolgenden nichts anderes ergibt. Insbesondere ist die Stielteil-Anordnung 204 in dem Femurknochenstumpf F verankert und weist einen distalen Abschnitt 222 auf, der zum Anbringen der Anordnung 202 von Brücken- bzw. Anbindungselementen vorgesehen ist. Ferner ist die Anbringungsöffnung 242 in einem proximalen Bereich 226 der Stielteil-Anordnung 204 angeordnet. Außerdem umfasst auch die Schenkelhals-Endoprothese 208 eine Schenkelhalsschraube 260 mit einem Gewinde 262, das in einem medialen Abschnitt der Schenkelhalsschraube 260 angeordnet ist, und einem Zylinderprofil 264, das in einem lateralen Abschnitt der Schenkelhalsschraube 260 angeordnet ist.

Die Schenkelhalsschraube 260 ist gemäß einer Ausrichtung der Anbringungsöffnung 242 in einem Winkel W in Bezug auf die Längsachse L der Stielteil-Anordnung 204 in den Schenkelhals S eingebracht. Die Schenkelhalsschraube 260 ist dabei entlang der Schenkelhalsachse H des Schenkelhalses S angeordnet. Eine Position der Anbringungsöffnung 242 in Bezug auf den Femurknochenstumpf F entspricht dabei zumindest annähernd einem Schnittpunkt der Schenkelhalsachse H und der Längsachse L der Stielteil-Anordnung 204.

Abweichend von dem Beispiel in Fig. 1 umgfasst die Stielteil-Anordnung 204 in einigen Beispielen einen Stielteil-Grundkörper 220, der sich zumindest im Wesentlichen über eine Gesamtlänge der Stielteilanordnung 204 erstreckt. Dabei umfasst die Stielteil-Anordnung 204 in einigen Beispielen abweichend von der Stielteil-Anordnung 104 in Fig. 1 kein Brückenmodul. Die Anbringungsöffnung 242 ist dabei in einem proximalen Bereich 226 des Stielteil-Grundkörpers 220 angeordnet. Die Stielteil-Anordnung 204 ist dabei zudem beispielsweise für einen vorliegenden Anwendungsfall ausgewählt unter mehreren Stielteilanordnungen 204, die bezüglich einer Länge verschieden sind. Zusätzlich oder alternativ ist die Stielteil-Anordnung 204 in weiteren Beispielen ausgewählt unter mehreren Stielteilanordnungen 204, die bezüglich einer Ausrichtung der Anbringungsöffnung 242 gemäß verschiedenen Winkeln W, einem Durchmesser der Anbringungsöffnung 242 und/oder einem Abstand der Anbringungsöffnung 242 von wenigstens einem aus einem proximalen Ende und einem distalen Ende der Stielteil-Anordnung 204 verschieden sind.

Die Femurkomponente 201 des transkutanen Implantats 200 umfasst in dem gezeigten Beispiel ein Femurimplantat-Set mit Stielteil-Anordnung 204 und Schenkelhals-Endoprothese 208. In einigen Beispielen umfasst dabei die Femurkomponente 201 weitere Bestandteile des transkutanen Implantats 200, die in Fig. 2 nicht dargestellt sind. Diese sind beispielsweise mit der Stielteil-Anordnun 204 starr verbunden und dienen zum Beispiel einem Verankern der Stielteil-Anordnung 204 in dem Femurknochenstumpf F und/oder dem Verbinden der Stielteil-Anordnung 204 mit der Anordnung 202 von Brücken- bzw. Anbindungselementen. In anderen als dem gezeigten Beispiel umfasst eine Femurkomponente eines implantierten transkutanen Implantats der hier vorgestellten Art eine Stielteil-Anordnung ohne daran angebrachte Schenkelhals-Endoprothese. Eine Schenkelhals-Endoprothese kann dabei beispielsweise zu einem späteren Zeitpunkt implantiert werden und so der Femurkomponente des transkutanen Implantats ergänzend hinzugefügt werden.

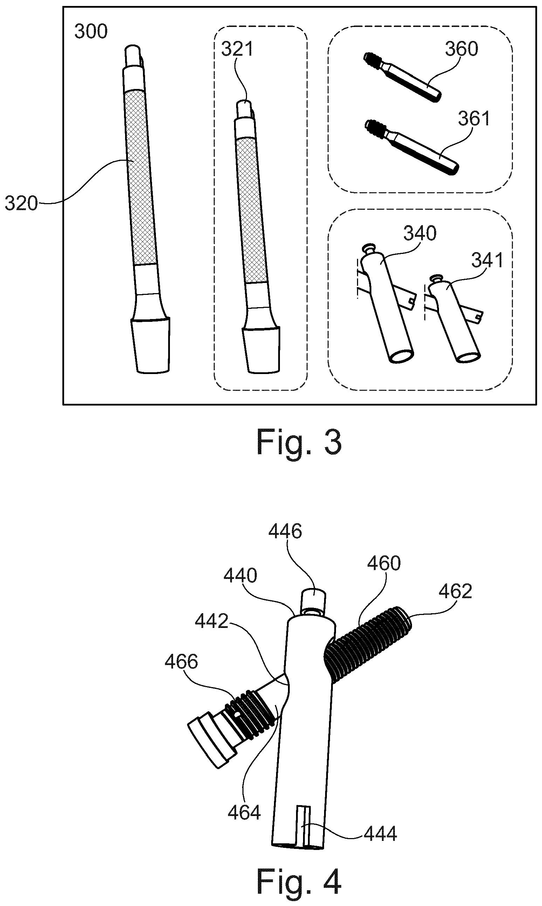

Fig. 3 zeigt schematisch und exemplarisch ein Femurimplantat-Set 300. Das Femurimplantat-Set 300 umfasst wenigstens ein Femurimplantat mit einem Stielteil-Grundkörper 320 gemäß einem der voranstehend im Zusammenhang mit Fig. 1 und 2 beschriebenen Beispiele.

In Beispielen des Femurimplantat-Sets 300, bei denen sich der Stielteil-Grundkörper 320 zumindest im Wesentlichen über eine Gesamtlänge der Stielteilanordnung erstreckt und an einem proximalen Ende eine Anbringungsöffnung aufweist, umfasst das Femurimplantat-Set 300 ferner wenigstens einen weiteren Stielteil-Grundkörper 321, der von dem Stielteil-Grundkörper 320 bezüglich einer Gesamtlänge, einer Ausrichtung der Anbringungsöffnung und/oder eines Durchmessers der Anbringungsöffnung verschieden ist. In weiteren Beispielen umfasst das Femurimplantat-Set 300 dabei wenigstens eine Schenkelhals-Endoprothese 360, 361, die zum Anbringen an dem wenigstens einen Stielteil-Grundkörper 320, 321 mittels der Anbringungsöffnung des wenigstens einen Stielteil-Grundkörpers 320, 321 vorgesehen ist. Beispielsweise umfasst das Femurimplantat-Set 300 mehrere Schenkelhals-Endoprothesen 360, 361, die voneinander verschieden sind bezüglich eines Durchmessers, einer Länge und/oder einer Funktion, beispielsweise zum Stützen eines Schenkelhalses und/oder zum Anbringen eines künstlichen Hüftgelenks an einem medialen Abschnitt der Schenkelhals-Endoprothese 360, 361.

In Beispielen des Femurimplantat-Sets 300, bei denen der Stielteil-Grundkörper 320 zur Verbindung mit einem Brückenmodul 340, 341 ausgebildet ist, das eine Anbringungsöffnung zum Anbringen einer Schenkelhals-Endoprothese aufweist, umfasst das Femurimplantat-Set 300 ferner wenigstens ferner wenigstens ein Brückenmodul 340. Dabei umfasst das Femurimplantat-Set 300 ferner wenigstens einen weiteren Stielteil-Grundkörper 321, der von dem Stielteil-Grundkörper 320 bezüglich einer Länge verschieden ist, wenigstens ein weiteres Brückenmodul 341, das von dem Brückenmodul 340 bezüglich eines Abstands der Anbringungsöffnung von dem Brückenmodul-Verbindungsabschnitt, einer Ausrichtung und/oder eines Durchmessers der Anbringungsöffnung verschieden ist, und/oder wenigstens eine Schenkelhals-Endoprothese 360, 361, die zum Anbringen an dem wenigstens einen Brückenmodul 340, 341 mittels der Anbringungsöffnung des Brückenmoduls 360, 361 vorgesehen ist.

Fig. 4 zeigt schematisch und exemplarisch ein Brückenmodul 440 und eine Schenkelhals-Endoprothese 460 gemäß einem weiteren Beispiel. Soweit sich aus dem Nachstehenden nichts anderes ergibt, gilt für das Brückenmodul 440 und die Schenkelhals-Endoprothese 460 das voranstehend im Zusammenhang mit einem Brückenmodul und einer Schenkelhals-Endoprothese Beschriebene entsprechend.

Auch das Brückenmodul 440 umfasst eine Anbringungsöffnung 442. Bei der gezeigten Anordnung ist die Schenkelhals-Endoprothese 460 in der Anbringungsöffnung 442 aufgenommen. Die Anbringungsöffnung 442 umfasst eine Bohrung, die sich durch das Brückenmodul 440 erstreckt und die schräg in Bezug auf eine bzw. quer zu einer Längserstreckungsrichtung des Brückenmoduls ausgerichtet ist. Das Brückenmodul umfasst zudem einen Brückenmodul-Verbindungsabschnitt 444 zum Verbinden des Brückenmoduls 440 mit einem Stielteil-Grundkörper. Bei dem Brückenmodul 440 weist der Brückenmodul-Verbindungsabschnitt 444 eine Nut auf.

Die Schenkelhals-Endoprothese 460 weist im medialen Abschnitt ein Gewinde 462 auf. Dabei handelt es sich beispielsweise um ein metrisches Gewinde, etwa zur Verschraubung mit einem Teil eines künstlichen Hüftgelenks. In einem lateral angrenzenden Abschnitt weist die Schenkelhals-Endoprothese 460 ein Zylinderprofil 464 auf. Das Zylinderprofil 464 dient beispielsweise zur drehbaren, formschlüssigen Aufnahme der Schenkelhals-Endoprothese 460 in der Anbringungsöffnung 442.

Abweichend von den vorhergehend beschriebenen Beispielen weist die Schenkelhals-Endoprothese 460 in einem Endabschnitt, der an das Zylinderprofil 464 lateral angrenzt, außerdem ein weiteres Gewinde 466 auf. Das weitere Gewinde 466 kann in einigen Beispielen vorgesehen sein, um im eingesetzten Zustand der Schenkelhals-Endoprothese 460 eine Verschraubung der Schenkelhals-Endoprothese 460 mit dem Brückenmodul 440 im Bereich der Anbringungsöffnung 442 und/oder mit Knochen, der das Brückenmodul 440 lateral umgibt, zu bewirken.

Das Brückenmodul 440 umfasst zudem in einigen Beispielen im proximalen Bereich eine zusätzliche Fixierungsbohrung und eine/n darin einbringbare/n Fixierungsschraube oder Fixierungsstift 446. Die zusätzliche Fixierungsbohrung ist beispielsweise parallel zur Längserstreckungsrichtung, insbesondere koaxial zur Längsachse des Brückenmoduls 440, angeordnet. Mittels der zusätzlichen Fixierungsbohrung und der/des darin einbringbaren Fixierungsschraube oder Fixierungsstifts 446 lässt sich eine Relativbewegung zwischen der Schenkelhals-Endoprothese 460 und der Stielteil-Anordnung nach einem Zusammenfügen sicher verhindern. Dies gilt besonders für Beispiele, bei denen die Schenkelhals-Endoprothese 460 kein Gewinde 466 aufweist, mittels dessen sie im eingesetzten Zustand mit dem Brückenmodul 440 im Bereich der Anbringungsöffnung 442 verschraubt ist.

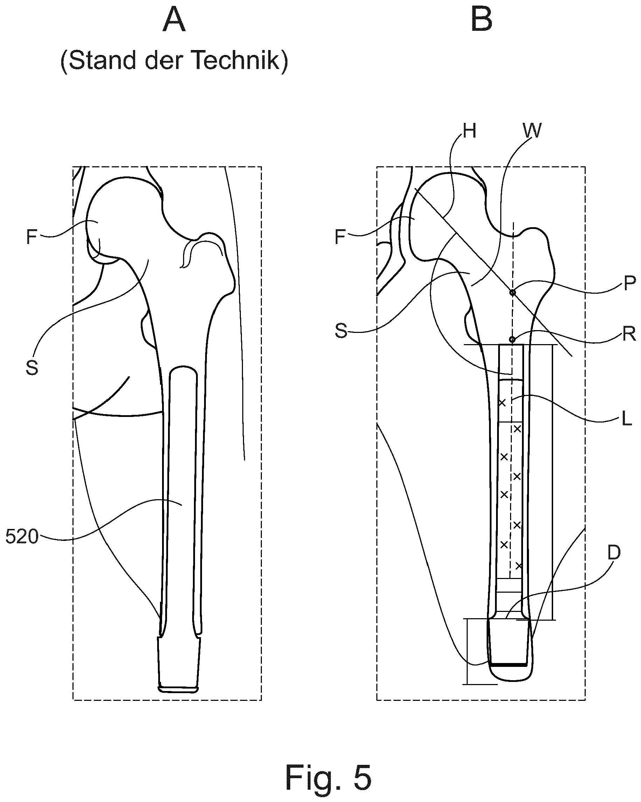

Fig. 5A zeigt schematisch und exemplarisch ein Stielteil 520 der eingangs beschriebenen, herkömmlichen Art, das in einem Femurknochenstumpf F verankert ist. Wie der Darstellung in Fig. 5A zu entnehmen ist, ist das Stielteil 520 bei einer vorgesehenen Länge des Femurknochenstumpfs F signifikant unterhalb des Schenkelhalses S angeordnet. Insbesondere erstreckt sich das Stielteil 520 in proximaler Richtung nicht bis zu einem geometrischen Schnittpunkt einer Längsachse des Stielteils 520 und einer Schenkelhalsachse des Schenkelhalses S, wobei der geometrische Schnittpunkt insbesondere dem Zentralpunkt des CCD-, d.h. Centrum-Collum-Diaphysen-, Winkels entspricht.

Fig. 5B zeigt schematisch einen Femurknochenstumpf F. Darin sind diagrammartig geometrische Merkmale eingezeichnet, die vorteilhaft als Bezugsgrößen für eine Auswahl, bzw. für ein Konfigurieren, einer Stielteil-Anordnung der vorgestellten Art dienen.

Dargestellt ist die Längsachse L der einzusetzenden Stielteil-Anordnung. Die Achse L entspricht dabei auch zumindest annähernd einer Achse des Femurschafts. Ferner ist die Schenkelhalsachse H gezeigt. Diese schneidet die Achse L in dem Punkt P. Die Achse L und die Schenkelhalsachse H verlaufen in einem Winkel W zueinander, der zumindest annähernd dem CCD-Winkel entspricht. Der Punkt P entspricht dabei zumindest annähernd dem Zentralpunkt des CCD-Winkels. Der Winkel W ist auf den unteren der beiden medialen Sektoren der sich schneidenden Achsen H, L bezogen. Außerdem ist der Resektionsbezugspunkt R für eine Resektion des Femurknochenstumpfs zum Bilden des distalen Endes D gemäß einer vorgesehenen Knochenschaftlänge, beispielsweise im Abstand von 170 Millimeter von dem Resektionsbezugspunkt R, gezeigt.

Anhand der vorgenannten Größen ist eine Stielteil-Anordnung der vorgestellten Art bezüglich einer Länge bevorzugt derart ausgewählt, bzw. durch geeignete Kombination eines Stielteil-Grundkörpers und wenigstens eines Brückenmoduls derart konfiguriert, dass nach einer vorgesehenen Verankerung der Stielteil-Anordnung in Bezug auf das distale Ende D des Femurknochenstumpfs F die Anbringungsöffnung in einem Bereich angeordnet ist, der sich möglichst nah dem Punkt P befindet. Die Stielteil-Anordnung ist zudem bevorzugt derart ausgewählt bzw. konfiguriert, dass die Anbringungsöffnung nach der Verankerung der Stielteil-Anordnung im Femurknochenstumpf F möglichst nah entlang der Schenkelhalsachse H ausgerichtet ist.

## Patentansprüche

1. Femurimplantat (104; 204) zum Bilden einer Femur-Komponente (201) eines transkutanen Implantats (200), insbesondere zur Verwendung mit einem Exo-Kniegelenk, wobei das Femurimplantat (104; 204) eine Stielteil-Anordnung (104; 204) umfasst, die in einem Femurknochenstumpf (F) verankerbar ist,
**dadurch gekennzeichnet, dass**
die Stielteil-Anordnung (104; 204) wenigstens eine Anbringungsöffnung (142; 242) aufweist, die in einem proximalen Bereich (140; 226) der Stielteil-Anordnung (104; 204) angeordnet ist und die dazu ausgebildet ist, ein Anbringen einer Schenkelhals-Endoprothese (108; 208) an der Stielteil-Anordnung (104; 204) zu gestatten, wenn die Stielteil-Anordnung (104; 204) in einem Femurknochenstumpf (F) verankert ist.

2. Femurimplantat nach Anspruch 1, wobei die Stielteil-Anordnung (104) wenigstens einen Stielteil-Grundkörper (120; 320, 321) und wenigstens ein Brückenmodul (140; 340, 341), das mit dem Stielteil-Grundkörper (120; 320, 321) in einer Längserstreckungsrichtung der Stielteil-Anordnung (104) verbindbar ist, umfasst.

3. Femurimplantat nach Anspruch 2, wobei die Anbringungsöffnung (142) in dem Brückenmodul (140; 340, 341) angeordnet ist.

4. Femurimplantat nach Anspruch 3, wobei das Brückenmodul (140; 340, 341) derart ausgebildet ist, dass die Anbringungsöffnung (142) in Bezug auf einen Schenkelhals (S) des Femurknochenstumpfs (F) in einem vorgesehenen Bereich angeordnet ist, wenn die Stielteil-Anordnung (104; 204) gemäß einer vorgesehenen Position in Bezug auf ein distales Ende des Femurknochenstumpfs verankert ist.

5. Femurimplantat nach einem der vorhergehenden Ansprüche, wobei die Stielteil-Anordnung (104; 204) in einer Längserstreckungsrichtung eine Länge im Bereich von 120 Millimeter bis 200 Millimeter, bevorzugt im Bereich von 140 Millimeter bis 180 Millimeter, aufweist.

6. Femurimplantat nach einem der vorhergehenden Ansprüche, wobei die Anbringungsöffnung (142; 242) ausgebildet ist zur Übertragung von Belastungskräften zwischen einer Schenkelhals-Endoprothese (108; 208) und der Stielteil-Anordnung (104; 204), wenn die Schenkelhals-Endoprothese (108; 208) mittels der Anbringungsöffnung (142; 242) an der Stielteil-Anordnung (104; 204) angebracht ist.

7. Femurimplantat nach einem der vorhergehenden Ansprüche, wobei die Anbringungsöffnung (142; 242) eine Bohrung umfasst, die sich durch die Stielteil-Anordnung (104; 204) erstreckt.

8. Femurimplantat nach einem der vorhergehenden Ansprüche, wobei die Anbringungsöffnung (142; 242) in Bezug auf eine Längsachse (L) der Stielteil-Anordnung (104; 204) derart ausgerichtet ist, dass sie ein Anbringen der Schenkelhals-Endoprothese (108; 208) an der Stielteil-Anordnung (104; 204) zumindest im Wesentlichen gemäß einem Schenkelhalswinkel (W) gestattet.

9. Femurimplantat nach einem der vorhergehenden Ansprüche, wobei die Anbringungsöffnung (142; 242) in Bezug auf die Längsachse (L) der Stielteil-Anordnung (104; 204) in einem Winkel (W) im Bereich von 130 Grad bis 150 Grad ausgerichtet ist.

10. Femurimplantat nach einem der vorhergehenden Ansprüche, wobei die Anbringungsöffnung (142; 242) zum Durchführen von wenigstens einem Teil der Schenkelhals-Endoprothese (108; 208) durch die Stielteil-Anordnung (104; 204), insbesondere von lateral, ausgebildet ist.

11. Femurimplantat nach Anspruch 10, wobei die Anbringungsöffnung (142; 242) einen Durchmesser im Bereich von 6 Millimeter bis 15 Millimeter, bevorzugt im Bereich von 8 Millimeter bis 12 Millimeter, aufweist.

12. Femurimplantat-Set (100; 204, 208; 300) umfassend wenigstens ein Femurimplantat (104; 204; 320) nach einem der vorhergehenden Ansprüche, wobei:
das Femurimplantat-Set (100; 204, 208; 300) ferner wenigstens eine Schenkelhals-Endoprothese (108; 208; 360, 361) umfasst, die mittels der Anbringungsöffnung (142; 242) der Stielteil-Anordnung (104; 204) des wenigstens einen Femurimplantats (104; 204) an der Stielteil-Anordnung (104; 204) anbringbar ist, und/oder
die Stielteil-Anordnung (104) des Femurimplantats (104) wenigstens einen Stielteil-Grundkörper (120; 320, 321) und wenigstens ein Brückenmodul (140; 340, 341), das mit dem Stielteil-Grundkörper (120; 320, 321) in einer Längserstreckungsrichtung der Stielteil-Anordnung (104) verbindbar ist, umfasst, wobei das Femurimplantat-Set (300) eine Mehrzahl von Brückenmodulen (340, 341) umfasst, die wahlweise mit dem wenigstens einen Stielteil-Grundkörper (320, 321) verbindbar sind und die jeweils eine Anbringungsöffnung (142) und einen Brückenmodul-Verbindungsabschnitt (144) zum Verbinden des Brückenmoduls (340, 341) mit dem Stielteil-Grundkörper (320, 321) aufweisen, wobei die Brückenmodule (340, 341) bezüglich wenigstens eines aus einem Abstand zwischen der Anbringungsöffnung (142) und dem Brückenmodul-Verbindungsabschnitt (144), einer Ausrichtung der Anbringungsöffnung (142) in Bezug auf eine Längsachse (L) der Stielteil-Anordnung und eines Durchmessers der Anbringungsöffnung (142) verschieden sind, und/oder
die Stielteil-Anordnung (104) des Femurimplantats wenigstens einen Stielteil-Grundkörper (120; 320, 321) und wenigstens ein Brückenmodul (140; 340, 341), das mit dem Stielteil-Grundkörper (120; 320, 321) in einer Längserstreckungsrichtung der Stielteil-Anordnung (104) verbindbar ist, umfasst, wobei das Femurimplantat-Set (300) eine Mehrzahl von Stielteil-Grundkörpern (320, 321) umfasst, die wahlweise mit dem wenigstens einen Brückenmodul (340, 341) verbindbar sind, wobei die Stielteil-Grundkörper (320, 321) bezüglich einer Größe in einer Längserstreckungsrichtung der Stielteil-Anordnung verschieden sind.

13. Femurimplantat-Set nach Anspruch 12, wobei das Femurimplantat-Set (100; 204, 208; 300) wenigstens eine Schenkelhals-Endoprothese (160; 260; 360, 361) umfasst, die eine Schenkelhalsschraube (160; 260) umfasst.

14. Femurimplantat-Set nach Anspruch 13, wobei die Anbringungsöffnung (142) eine Bohrung umfasst und die Schenkelhalsschraube (160; 260) in einem medialen Abschnitt ein Gewinde (162; 262) und in einem lateralen Abschnitt ein Zylinderprofil (164; 264) aufweist.

15. Femurimplantat-Set nach einem der Ansprüche 12 bis 14, wobei das Femurimplantat-Set (300) wenigstens eine Schenkelhals-Endoprothese (360, 361) umfasst, die zum Verstärken eines Schenkelhalses (S) und/oder zum Anbringen wenigstens eines Teils einer Hüft-Endoprothese an der Schenkelhals-Endoprothese (360, 361) ausgebildet ist, wenn das Femurimplantat in einem Femurknochenstumpf (F) verankert ist.

## Geänderte Patentansprüche

### Geänderte Patentansprüche gemäss Regel 137(2) EPÜ.

1. Femurimplantat-Set (300) umfassend wenigstens ein Femurimplantat (104; 204) zum Bilden einer Femur-Komponente (201) eines transkutanen Implantats (200), insbesondere zur Verwendung mit einem Exo-Kniegelenk, wobei das Femurimplantat (104; 204) eine Stielteil-Anordnung (104; 204) umfasst, die in einem Femurknochenstumpf (F) verankerbar ist und die wenigstens eine Anbringungsöffnung (142; 242) aufweist, die in einem proximalen Bereich (140; 226) der Stielteil-Anordnung (104; 204) angeordnet ist und die dazu ausgebildet ist, ein Anbringen einer Schenkelhals-Endoprothese (108; 208) an der Stielteil-Anordnung (104; 204) zu gestatten, wenn die Stielteil-Anordnung (104; 204) in einem Femurknochenstumpf (F) verankert ist, wobei die Stielteil-Anordnung (104) wenigstens einen Stielteil-Grundkörper (120; 320, 321) und wenigstens ein Brückenmodul (140; 340, 341), das mit dem Stielteil-Grundkörper (120; 320, 321) in einer Längserstreckungsrichtung der Stielteil-Anordnung (104) verbindbar ist, umfasst,
wobei das Femurimplantat-Set (300) umfasst:
eine Mehrzahl von Brückenmodulen (340, 341), die wahlweise mit dem wenigstens einen Stielteil-Grundkörper (320, 321) verbindbar sind und die jeweils eine Anbringungsöffnung (142) und einen Brückenmodul-Verbindungsabschnitt (144) zum Verbinden des Brückenmoduls (340, 341) mit dem Stielteil-Grundkörper (320, 321) aufweisen, wobei die Brückenmodule (340, 341) bezüglich wenigstens eines aus einem Abstand zwischen der Anbringungsöffnung (142) und dem Brückenmodul-Verbindungsabschnitt (144), einer Ausrichtung der Anbringungsöffnung (142) in Bezug auf eine Längsachse (L) der Stielteil-Anordnung und eines Durchmessers der Anbringungsöffnung (142) verschieden sind, und/oder
eine Mehrzahl von Stielteil-Grundkörpern (320, 321), die wahlweise mit dem wenigstens einen Brückenmodul (340, 341) verbindbar sind, wobei die Stielteil-Grundkörper (320, 321) bezüglich einer Größe in einer Längserstreckungsrichtung der Stielteil-Anordnung verschieden sind.

2. Femurimplantat-Set nach Anspruch 1, wobei das wenigstens eine Brückenmodul (140; 340, 341) derart ausgebildet ist, dass die Anbringungsöffnung (142) in Bezug auf einen Schenkelhals (S) des Femurknochenstumpfs (F) in einem vorgesehenen Bereich angeordnet ist, wenn die Stielteil-Anordnung (104; 204) gemäß einer vorgesehenen Position in Bezug auf ein distales Ende des Femurknochenstumpfs verankert ist.

3. Femurimplantat-Set nach einem der vorhergehenden Ansprüche, wobei die Stielteil-Anordnung (104; 204) in einer Längserstreckungsrichtung eine Länge im Bereich von 120 Millimeter bis 200 Millimeter, bevorzugt im Bereich von 140 Millimeter bis 180 Millimeter, aufweist.

4. Femurimplantat-Set nach einem der vorhergehenden Ansprüche, wobei die Anbringungsöffnung (142; 242) ausgebildet ist zur Übertragung von Belastungskräften zwischen einer Schenkelhals-Endoprothese (108; 208) und der Stielteil-Anordnung (104; 204), wenn die Schenkelhals-Endoprothese (108; 208) mittels der Anbringungsöffnung (142; 242) an der Stielteil-Anordnung (104; 204) angebracht ist.

5. Femurimplantat-Set nach einem der vorhergehenden Ansprüche, wobei die Anbringungsöffnung (142; 242) eine Bohrung umfasst, die sich durch die Stielteil-Anordnung (104; 204) erstreckt.

6. Femurimplantat-Set nach einem der vorhergehenden Ansprüche, wobei die Anbringungsöffnung (142; 242) in Bezug auf eine Längsachse (L) der Stielteil-Anordnung (104; 204) derart ausgerichtet ist, dass sie ein Anbringen der Schenkelhals-Endoprothese (108; 208) an der Stielteil-Anordnung (104; 204) zumindest im Wesentlichen gemäß einem Schenkelhalswinkel (W) gestattet.

7. Femurimplantat-Set nach einem der vorhergehenden Ansprüche, wobei die Anbringungsöffnung (142; 242) in Bezug auf die Längsachse (L) der Stielteil-Anordnung (104; 204) in einem Winkel (W) im Bereich von 130 Grad bis 150 Grad ausgerichtet ist.

8. Femurimplantat-Set nach einem der vorhergehenden Ansprüche, wobei die Anbringungsöffnung (142; 242) zum Durchführen von wenigstens einem Teil der Schenkelhals-Endoprothese (108; 208) durch die Stielteil-Anordnung (104; 204), insbesondere von lateral, ausgebildet ist.

9. Femurimplantat-Set nach Anspruch 8, wobei die Anbringungsöffnung (142; 242) einen Durchmesser im Bereich von 6 Millimeter bis 15 Millimeter, bevorzugt im Bereich von 8 Millimeter bis 12 Millimeter, aufweist.

10. Femurimplantat-Set nach einem der vorhergehenden Ansprüche, wobei das Femurimplantat-Set (100; 204, 208; 300) ferner wenigstens eine Schenkelhals-Endoprothese (160; 260; 360, 361) umfasst, die eine Schenkelhalsschraube (160; 260) umfasst.

11. Femurimplantat-Set nach Anspruch 10, wobei die Anbringungsöffnung (142) eine Bohrung umfasst und die Schenkelhalsschraube (160; 260) in einem medialen Abschnitt ein Gewinde (162; 262) und in einem lateralen Abschnitt ein Zylinderprofil (164; 264) aufweist.

12. Femurimplantat-Set nach einem der vorhergehenden Ansprüche, wobei das Femurimplantat-Set (300) wenigstens eine Schenkelhals-Endoprothese (360, 361) umfasst, die zum Verstärken eines Schenkelhalses (S) und/oder zum Anbringen wenigstens eines Teils einer Hüft-Endoprothese an der Schenkelhals-Endoprothese (360, 361) ausgebildet ist, wenn das Femurimplantat in einem Femurknochenstumpf (F) verankert ist.
